# EUROPÄISCHE PATENTSCHRIFT

(11) **EP 0 730 031 B1**
(45) Veröffentlichungstag und Bekanntmachung des Hinweises auf die Patenterteilung: **09.06.1999**
(21) Anmeldenummer: 96101094.9
(22) Anmeldetag: 26.01.1996
(51) Int. Cl.: C12P 5/02, B09B 3/00, C12M 1/107

(54) **Verfahren zum mikrobiellen Abbau organisch belasteter Substrate**
Process for the microbial degradation of organic materials containing substrates
Procédé pour la dégradation microbienne de substrats chargés de matières organiques

(30) Priorität: 02.03.1995 DE 19507258
(43) Veröffentlichungstag der Anmeldung: 04.09.1996
(73) Patentinhaber: SCHWARTING- UHDE GmbH, D-24941 Flensburg (DE); FRAUNHOFER-GESELLSCHAFT ZUR FÖRDERUNG DER ANGEWANDTEN FORSCHUNG E.V., 80636 München (DE)
(72) Erfinder: Schwarting, Norbert, D-88085 Langenargen (DE); Schmid-Staiger, Ulrike, Dr. rer. nat., D-72654 Neckartenzlingen (DE); Trösch, Walter, Dr. rer. nat.-Biologe, D-70329 Stuttgart (DE)
(74) Vertreter: Engelhardt, Guido, Dipl.-Ing.

(56) Entgegenhaltungen:
- DE-A- 4 114 667
- US-A- 4 185 680
- DATABASE WPI Section Ch, Week 9239 Derwent Publications Ltd., London, GB; Class D15, AN 92-320973 XP002014572 & JP 04 227 899 A (EBARA INFILCO KK) , 17.August 1992

## Beschreibung

Die Erfindung bezieht sich auf ein Verfahren zum mikrobiellen Abbau organisch belasteter Substrate, insbesondere von Großküchenabfällen und Biomüll, wobei der Abbau in mehreren Stufen erfolgt und in einem Reaktor als Methanisierungsstufe vorgenommen wird.

Der große Nachteil biologischer Verfahren allgemein, so auch speziell für Verfahren der Vergärung, besteht in ihrer "verfahrenstechnische Omnipotenz". Organische Substanz vergärt fast in jedem Behältnis, wenn Sauerstoffausschluß gewährleistet ist. Deshalb gibt es eine Reihe von Verfahren, die für die Vergärung von organischen Feststoffsubstraten angewandt werden. Die Zusammensetzung des Substrates nämlich Restmüll, getrennt gesammelte organische Hausmüllfraktion, Großküchenabfälle und dgl. bestimmt im wesentlichen die optimale Verfahrensvariante.

Durch die DE-C2-32 08 977 ist ein derartiges Verfahren bekannt. Mit diesem Verfahren werden organische Verbindungen und Feststoffe abgebaut, wie sie in schwer abbaubaren Substraten, wie z. B. Gülle oder Klärschlamm oder leicht abbaubaren Substraten, wie z. B. Abwasser aus der Kartoffel-, der Stärke-und der Alkoholindustrie, vorliegen. Nach diesem Verfahren werden zunächst die leichter und mittelschwer abbaubaren Betandteile methanisiert. Für diese Fraktion organischer Verbindungen ist die Methanbildung der limitierende Schritt des anaeroben Abbaus. Deswegen führen zu hohe Substratraumbelastungen zu einer Versäuerung des Reaktorinhaltes, d. h. die maximal mögliche Raumbelastung für organische Abfälle wird von der Konzentration an leicht abbaubaren Bestandteilen im Abfall bzw. Reststoff bestimmt. Überschreitet man diese Grenze, versauert der Reaktor und die Methanbildung kommt zum Erliegen.

Innerhalb dieser Raumbelastung werden jedoch die schwerer abbaubaren Bestandteile nicht abgebaut. Bei dem bekannten Verfahren ist demnach der anaerobe Abbau auf die leicht bis mittelschwer abbaubaren Bestandteile und somit bei organischen Abfällen auf ca. 50 bis 60 % beschränkt.

Aufgabe der Erfindung ist es daher, das Verfahren der eingangs genannten Gattung in der Weise zu verbessern, daß Rest- und Abfallstoffe mit hoher Konzentration an Feststoffen biologisch bis auf eine sehr kleine Restmenge abzubauen sind, so daß bei einer Deponierung der noch verbleibenden Reste keine gasförmigen und flüssigen Emmisionen verursacht werden. Außerdem soll die in den abzubauenden Substraten enthaltene Energie optimal ausgenutzt werden können und der dazu erforderliche Bauaufwand soll gering gehalten werden, so daß bei einfacher und störungsfreier Betriebsweise ein hoher wirtschaftlicher Wirkungsgrad gegeben ist.

Gemäß der Erfindung wird dies bei dem Verfahren zum mikrobiellen Abbau organischer belasteter Substrate der vorgenannten Art dadurch erreicht, daß die dem Reaktor der Methanisierungsstufe entnommenen organischen Reststoffe einer z. B. durch eine Zentrifuge gebildeten Trennstufe zugeführt werden, in der die organischen Reststoffe aufkonzentriert werden und die in diesen enthaltene Flüssigkeit um mindestens 50 %, höchstens jedoch um 85 %, redzuiert und abgeführt wird, und daß der Trennstufe ein zweiter Reaktor als zweite Methanisierungsstufe zugeordnet ist, dem die der Trennstufe entnommenen aufkonzentrierten organischen Reststoffe zugeführt werden.

Zweckmäßig ist es hierbei des weiteren, dem zweiten Reaktor eine zweite Trennstufe zuzuordnen, in der die dem zweiten Reaktor entnommenen organischen Reststoffe erneut aufkonzentriert werden und die in diesem enthaltene Feuchtigkeit um mindestens 50 % reduziert und abgeführt wird.

Die der zweiten Trennstufe entnommenen organischen Reststoffe können problemlos kompostiert werden, auch kann die Aufkonzentrierung und Methanisierung bei mesophilen oder thermophilen Temperaturen erfolgen. Ferner ist es angebracht, um die Gasausbeute zu erhöhen, dem zweiten Reaktor zur enzymatischen Behandlung der der vorgeschalteten Trennstufe entnommenen aufkonzentrierten organischen Reststoffe lytisches Enzym, Alkalase, alkalische Cellulase oder Protease zuzugeben.

Nach dem erfindungsgemäßen Verfahren werden somit nach der Versäuerung und Methanisierung der leicht bis mittelschwer abbaubaren Bestandteile das Substrat und damit die schwer abbaubaren Bestandteile aufkonzentriert und einer zusätzlichen anaeroben Stufe mit einer hohen Konzentration zugeführt, in der ein großer Teil der schwer abbaubaren Anteile demnach abgebaut wird. Dadurch werden für solche Abfälle Abbauraten von 80 % - 90 % ermöglicht.

Durch die Erhöhung der Substratkonzentration wird somit die Abbaurate wesentlich erhöht. Werden nämlich die Feststoffe, die durch die einstufige Vergärung nicht abgebaut wurden, aufkonzentriert, von 1,0 bis 1,5 % organischer Trockensubstanzgehalt auf 7 %, und erneut einer Vergärung unterworfen, so werden nochmals 55 % der organischen Trockensubstanz zu Biogas umgesetzt. Erfolgt kein Aufkonzentrierungsschritt, würden bei gleicher Verweilzeit nur ca. 25 % der organischen Trockensubstanz abgebaut. Abbauraten von 2,8 kg OTS/m³ Tag werden nochmals erreicht. Bei diesem Abbau der langsam / schwerabbaubaren Substanzen ist die Hydrolyse der limitierende Schritt der Vergärung, deshalb kann eine hohe Substratkonzentration eingesetzt werden, um die Abbaurate zu verbessern, ohne Gafahr zu laufen, daß der Reaktor versäuert.

Insgesamt können mittels der beiden Vergärungsschritte in den beiden Reaktoren bis zu 90 % der eingesetzten organischen Trockensubstanz abgebaut werden.

Mittels des erfindungsgemäßen Verfahrens ist es demnach möglich, organisch belastete Substrate in einem außerordentlich hohen Maße abzubauen, und zwar auf eine sehr kostengünstige Art. Die in den Substraten enthaltene Energie kann dabei optimal ausgenutzt werden, auch sind während des Abbaus der Substrate keine Betriebsstörungen, wie diese z. B. bei deren Verbrennung auftreten können, zu befürchten, und bei einer Deponierung der Reststoffe sind kein Sickerwasser und keine gasförmigen Emmisionen in Kauf zu nehmen. Das erfindungsgemäße Verfahren ermöglicht demnach auf wirtschaftliche und betriebssichere Weise einen biologischen Abbau von organisch belasteten Substraten.

Naturstoffabfälle enthalten immer Anteile, die schnell umwandelbar sind, wie Stärke, Zucker, Eiweißfraktionen, Anteile, die langsam abbaubar sind, wie Cellulose, einige Fettfraktionen, Feststoffe aus Naturstoffen allgemein und dgl. sowie Anteile, die extrem langsam abbaubar sind, wie Holz und Lignocellulose. In diesen Substraten sind die Anteile schnell, langsam und extrem langsam umwandelbarer Naturstoffe deutlich verschieden. So ist beispielsweise der Anteil extrem schwer abbaubarer Anteile in den Großküchenabfällen sehr gering, im Restmüll dagegen sehr hoch. Zusätzlich schwanken die Anteile über die Woche und erst recht übers Jahr.

Die maximale Zulaufraumbelastung für einen Anaerobreaktor - soll er nicht versäuern - wird bestimmt durch den Anteil von schnell abbaubaren, gelösten und festen Verbindungen im Gärsubstrat. Wegen der unterschiedlichen Wachstumsgeschwindigkeit zwischen versäuernden (schnell)- und methanbildenden (langsam)-Bakterien wird verfahrenstechnisch hier die Methanisierung zum geschwindigkeitsbestimmenen Schritt. Aus eigenen Untersuchungen von Großküchenabfällen ermittelt, liegt die maximale Raumbelastung für schnell abbaubare Fraktionen im Abfall bei ca. 4 bis 6 kg 0TS/m³x d für Suspensionen mit ca. 10 % TS.

Aus einem Vergleich der Feststofffraktion vor und nach einer Methansierung geht hervor, daß es vorteilhaft ist, die Feststofffraktion, die nach der Versäuerungsreaktion übrigbleibt, zusammen mit den Säuren der Methanisierung zuzuführen. Unter den niedrigen Partialdrücken für Wasserstoff im Methanreaktor ist beispielsweise die Cellulosehydrolyse nicht unterdrückt, was im wesentlichen einen hohen Feststoffabbau ermöglicht.

Die Feststoffabtrennung vor der Methanisierung, wie vorgeschlagen, reduziert den Gesamtwirkungsgrad der Bioabfallvergärung.

Besteht das Eingangssubstrat für die Vergärung - wie im Restmüll - hauptsächlich aus schwer bis extrem schwer abbaubaren Fraktionen, ist die Feststoffhydrolyse der geschwindigkeitsbestimmende Schritt der anaeroben Umwandlung. Die Raumbelastung für einen Methanisierungsreaktor kann, analog zur Versäuerungsrate einem schnell versäuernden Eigangssubstrat, porportional zum geringeren Feststoffabbaugrad erhöht werden. Eine höhere Verweilzeit führt zu keiner wesentlichen Verbesserung des Abbaus organischer Feststoffe.

Die von Monod in seinem Wachstumsmodell vorgeschlagene Abhängigkeit der Wachstumsgeschwindigkeit eines Mikroorganismus von der Substratkonzentration (limitierend) beschreibt mit der Sättigungskonstante (ks) jede Substratkonzentration, bei welcher halbmaximales Wachstum erreicht wird und damit die Affinität des limitierenden Substrats zum Mikroorganismus. Kleine Sättigungskonstanten stehen für hohe Affinität, große Sättigungskonstanten für niedrige Substrataffinität. Im anaeroben Bereich entspricht die Wachstumsrate weitgehend der Abbaurate (wachstumsgekoppelte Produktbildung).

Stehen die leicht löslichen und die gelösten Betandteile der zu vergärenden Masse, die auch schnell methansiert werden, für Substrate mit hoher Affinität zu Bakterien, die sie methanisieren (kleine ks-Werte), dann verläuft der Abbau über einen weiten Konzentrationsbereich mit maximaler Geschwindigieit ab (formal 0. Ordnung). Erst bei sehr kleinen Konzentrationen (< 1 g/l) wird die Substratkonzentration wachstums- und abbaulimitierend.

Dies gilt jedoch nicht für die schwer abzubauenden Feststofffraktionen. Ihre Affinität zur methanbildenden Biozoenose (formal betrachtet) ist niedrig, ihre Sättigungskonstante hoch und dies bedeutet, daß die Abbaugeschwindigkeit über einen weiten Konzentrationsbereich von der Substratkonzentration abhängig ist, d. h. formal 1. Ordung entspricht. Diese Abbaugeschwindigkeit kann, wenn die hier getroffene Annahme sich als tragfähig erweist, durch Erhöhung der Konzentration des limitierenden Substrates erhöht werden.

Vergleicht man eine Nachgärung ohne Aufkonzentration der abtrennbaren Bestandteile (1,5 % 0TS) mit einer Vergärung, bei welcher durch Abtrennung der wässerigen Phase die organische Trockensubstanz (0TS) von 1,5 % erhöht wurde, ergeben sich folgende Tabellenwerte:

| | Verweilzeit (Tage) | Raumbelastung (kg 0TS/m³x d) | 0TS-Reduktion % | Abbaurate (kg 0TS/m³x d) |
|---|---|---|---|---|
| 55 °C₁ | 14,0 | 5,0 | 55 | 2,80 |
| 55 °C₂ | 18 | 0,8 | 30 | 0,25 |

| | | | | |
|---|---|---|---|---|
| 1) Reststoffe nach einstufiger Vergärung aufkonzentriert auf 7 % 0TS | | | | |
| 2) Reststoffe nach einstufiger Vergärung nicht aufkonzentriert, = 1,5 % 0TS | | | | |

Bei einer einfachen Nachgärung kann man immerhin bei kleiner Raumbleastung noch ca. 30 % der Organik zu Biogas umwandeln. Allerdings ist die Abbaugeschwindigkeit mit 0,25 Kg 0TS/m³x d bescheiden. Konzentriert man die verbleibende organische Masse auf 7 % 0TS auf, kann man immerhin 55 % der Organik methanisieren und dies bei 10-facher Rate = Abbaugeschwindigkeit (2,5 kg 0TS/m³x d).

Der Gesamtabbau hat sich nunmehr durch zwei Methanstufen auf 70 % + 55 % von der verbleibenden Masse (30 %) auf 86 % Gesamtwirkungsgrad erhöht. Das für eine zweite Vergärungsstufe von Großküchenabfällen benötigte Volumen liegt etwa bei ¼ oder 1/5 des Volumens der ersten Stufe.

In der Zeichnung ist in schematischer Darstellung der erfindungsgemäße Verfahrensablauf beim Abbau organisch belasteter Substrate dargetellt und nachfolgend im einzelnen erläutert. Hierbei zeigt:
- Figur 1: eine Methanisierungsstufe, der eine Trennstufe sowie eine zweite Methanisierungsstufe nachgeschaltet sind,
- Figur 2: die erste Methanisierungsstufe mit Trennstufe und zweiter Methanisierungsstufe nach Figur 1 mit dieser nachgeschalteter zweiter Trennstufe,
und

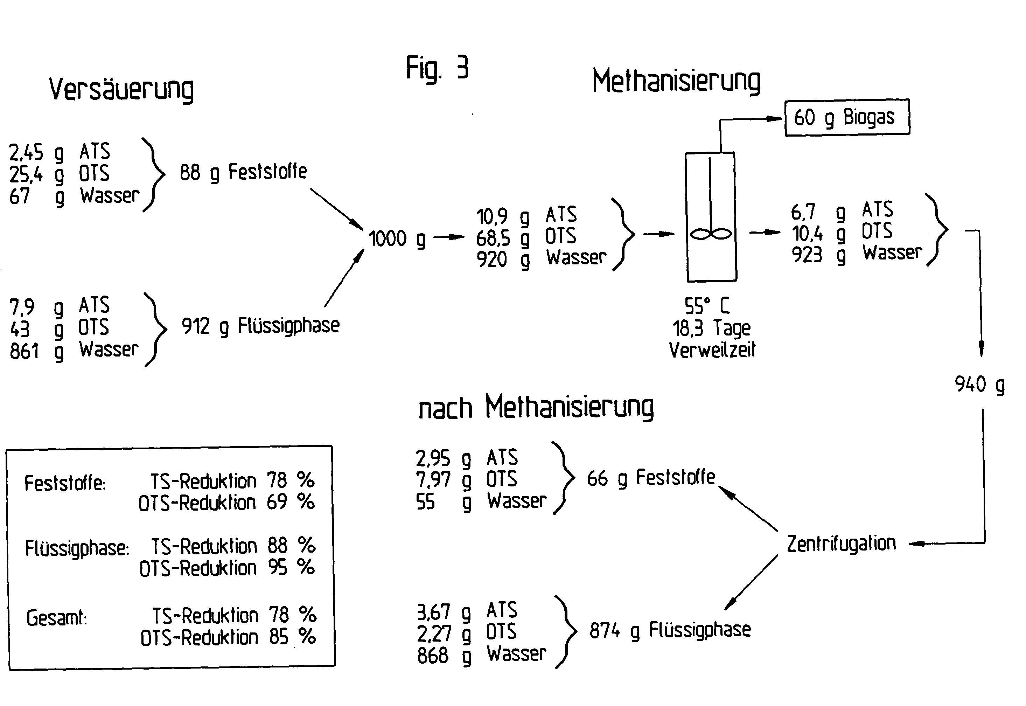

- Figur 3: eine Massenbilanz thermophiler Vergärung von Großküchenabfällen.

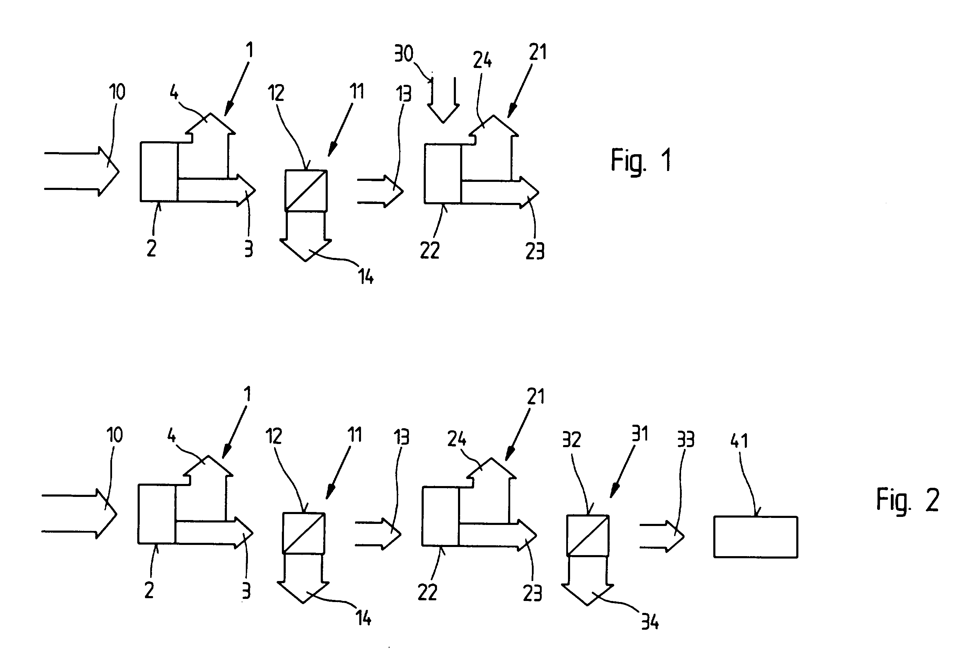

Nach Figur 1 wird der z. B. in einer Großküche anfallende Restmüll 10 einer Methanisierungsstufe 1 zugeführt, die aus einem Reaktor 2 besteht. In dem Reaktor 2 wird der Restmüll 10 durch Vergärung in organische Reststoffe 3 sowie Biogas 4, das einem Heizkraftwerk zugeführt werden kann, aufgespalten. Bis zu 65 % können hierbei als leicht abbaubare Fraktion in Form von Biogas 4 gewonnen werden, als schwer abbaubare Fraktion sind dem Reaktor 2 somit etwa 35 % des eingegebenen Restmülls 10 als organische Reststoffe 3 zu entnehmen.

Um die organischen Reststoffe 3 weiter abzubauen, ist der Methanisierungsstufe 1 eine Trennstufe 11 nachgeschaltet, die z. B. als Zentrifuge 12 ausgebildet ist. In der Trennstufe 11 werden dieorganischen Reststoffe 3 aufkonzentriert, in dem die in diesen enthaltene Flüssigkeit um mindestens 50 % reduziert wird. Der Trennstufe 11 ist somit eine Trockensubstanz 13 entnehmbar, die in der Trennstufe 11 aus den organischen Reststoffen 3 ausgeschiedene Flüssigkeit 14 wird gegebenenfalls in einen Wasserkreislauf eingespeist.

Des weiteren ist vorgesehen, der Trennstufe 13 einer zweiten Methanisierungsstufe 21, die ebenfalls aus einem Reaktor 22 besteht, zuzuführen. Der Trockensubstanz 13 wird in der zweiten Methanisierungsstufe 21 wiederum Biogas 24, und zwar bis zu 60 % entzogen, das energetisch zu nutzen ist, die verbleibende Trockensubstanz 23 kann in geeigneter Weise verwertet werden.

Bei der Weiterbildung nach Figur 2 ist der zweiten Methanisierungsstufe 21 eine zweite Trennstufe 31, die als Zentrifuge 32 ausgebildet ist, zugeordnet, in der die Trockensubstanz 23 wiederum aufkonzentriert und die in dieser enthaltene Flüssigkeit um mindestens 50 % reduziert wird. Die in der zweiten Trennstufe 31 verbleibende Trockensubstanz 33 kann einer Kompostanlage 41, die entnommene Flüssigkeit 34 einem Wasserkreislauf zugeführt weren.

Um die Gasausbeute in der zweiten Methanisierungsstufe zu steigern, können den in den Reaktor 22 eingebrachten organischen Reststoffen 13 Enzyme 30 oder vergleichbare Stoffe beigegeben werden.

## Patentansprüche

1. Verfahren zum mikrobiellen Abbau organisch belasteter Substrate (10), insbesondere von Großküchenabfällen und Biomüll, wobei der Abbau in mehreren Stufen erfolgt und in einem Reaktor (2) als Methanisierungsstufe (1) vorgenommen wird,
**dadurch gekennzeichnet,**
daß die dem Reaktor (2) der Methanisierungsstufe (1) entnommenen organischen Reststoffe (3) einer z.B. durch eine Zentrifuge (12) gebildeten Trennstufe (11) zugeführt werden, in der die organischen Reststoffe (3) aufkonzentriert werden und die in diesen enthaltene Flüssigkeit (14) um mindestens 50 %, höchstens jedoch um 85 %, reduziert und abgeführt wird, und daß der Trennstufe (11) ein zweiter Reaktor (22) als zweite Methanisierungsstufe (21) zugeordnet ist, dem die der Trennstufe (11) entnommenen aufkonzentrierten organischen Reststoffe (13) zugeführt werden.

2. Verfahren nach Anspruch 1,
**dadurch gekennzeichnet,**
daß dem zweiten Reaktor (22) eine zweite Trennstufe (31) zugeordnet ist, in der die dem zweiten Reaktor (22) entnommenen organischen Reststoffe (23) aufkonzentriert werden und die in diesen enthaltene Flüssigkeit (24) um mindestens 50 %, reduziert und abgeführt wird.

3. Verfahren nach Anspruch 1 oder 2,
**dadurch gekennzeichnet,**
daß die der zweiten Trennstufe (31) entnommenen organischen Reststoffe (33) kompostiert werden.

4. Verfahren nach einem oder mehreren der Ansprüche 1 bis 3,
**dadurch gekennzeichnet,**
daß die Aufkonzentrierung und Methanisierung in der zweiten Trennstufe (31) bei mesophilen oder thermophilen Temperaturen erfolgt.

5. Verfahren nach einem oder mehreren der Ansprüche 1 bis 4,
**dadurch gekennzeichnet**
daß dem zweiten Reaktor (22) zur enzymatischen Behandlung der der vorgeschalteten Trennstufe (11) entnommenen aufkonzentrierten organischen Reststoffe (13) lytisches Enzym, Alkalose, alkalische Cellulase oder Protease zugegeben wird.

## Claims

1. A process for the microbial degradation of substrates (10) containing organic materials, in particular of waste from large kitchens and bio waste, in which the degradation takes place over several stages and occurs in a reactor (2) as a methanation stage (1),
**characterised in that**,
the organic residues (3) removed from the reactor (2) of the methanation stage (1) are supplied to a separation stage (11) formed, for example, by a centrifuge (12) in which the concentration of the organic residues (3) is increased and the liquid (14) contained in the residues (3) is reduced by at least 50 % and no more than 85 % before being removed, and that a second reactor (22) is allocated to the separation stage (11) in the form of a second methanation stage (21) to which the concentrated organic residues (13) removed from the separation stage (11) are supplied.

2. The process in accordance with Claim 1,
**characterised in that,**
a second separation stage (31) is allocated to the second reactor (22), in which the concentration of the organic residues (23) removed from the second reactor (22) is increased and the liquid (24) contained in the residues (23) is reduced by at least 50 % before being removed.

3. The process in accordance with Claim 1 or 2,
**characterised in that,**
the organic residues (23) removed from the second separation stage (31) are composted.

4. The process in accordance with one or more of Claims 1 to 3,
**characterised in that,**
the concentration and methanation in the second separation stage (31) take place at mesophilic or thermophilic temperatures.

5. The process in accordance with one or more of Claims 1 to 4,
**characterised in that,**
lytic enzyme, alkalose, alkaline cellulase or protease are added to the second reactor (22) for enzyme treatment of the concentrated organic residues (13) removed from the upstream separation stage (11).

## Revendications

1. Procédé pour la dégradation microbienne de substrats chargés de matières organiques (10), notamment des déchets de grandes cuisines et des déchets biologiques, la dégradation se faisant en plusieurs phases en tant qu'étage de méthanisation (1) dans un réacteur (2),
caractérisé en ce que
les substrats organiques restants (3) prélevés du réacteur (2) de l'étage de méthanisation (1) sont acheminés vers un étage de séparation (11) formé par exemple par une centrifuge (12), dans lequel les substrats organiques restants (3) sont concentrés et où le liquide (14) contenu dans ceux-ci est réduit d'au moins 50 % et au maximum de 85 %, puis évacué, et en ce qu'il est assigné à l'étage de séparation (11), un deuxième réacteur (22) en tant que deuxième étage de méthanisation (21) qui reçoit les substrats organiques restants concentrés (13), prélevés de l'étage de séparation (11).

2. Procédé d'après la revendication 1,
caractérisé en ce que,
au deuxième réacteur (22), il est assigné un deuxième étage de séparation (31), dans lequel sont concentrés les substrats organiques restants (23) prélevés du deuxième réacteur (22) et où le liquide (24) contenu dans ceux-ci est réduit d'au moins 50 %, puis évacué.

3. Procédé d'après la revendication 1 ou 2,
caractérisé en ce que
les substrats organiques restants (33) prélevés du deuxième étage de séparation (31) sont compostés.

4. Procédé d'après une des revendications 1 à 3,
caractérisé en ce que
dans le deuxième étage de séparation (31), la concentration et la méthanisation se font à des températures mésophiles et thermophiles.

5. Procédé d'après une des revendications 1 à 4,
caractérisé en ce que
pour le traitement enzymatique des substrats organiques restants concentrés (13) prélevés de l'étage de séparation (11) précédent, il est ajouté au deuxième réacteur (22) de l'enzyme lytique, de l'alcalose, de la cellulase alcaline ou de la protase.
